# EUROPÄISCHE PATENTANMELDUNG

(11) **EP 4 435 510 A1**
(43) Veröffentlichungstag der Anmeldung: **25.09.2024**
(21) Anmeldenummer: 23163365.2
(22) Anmeldetag: 22.03.2023
(51) Int. Cl.: G03B 7/00, G05B 19/00, G16H 20/17, G16H 40/40

(54) **VERFAHREN ZUR AUTOMATISIERTEN ENDABFERTIGUNG VON INFUSIONSPUMPEN UND ZUGEHÖRIGES ENDABFERTIGUNGSSYSTEM**

(71) Anmelder: B. Braun Melsungen AG, 34212 Melsungen (DE)
(72) Erfinder: Blume, Markus, 33014 Bad Driburg-Dringenberg (DE); Breidenstein, Tim, 34281 Gudensberg (DE); Haemmerle, Simon, 34119 Kassel (DE); Herwig, Dominic, 37214 Witzenhausen (DE); Mueller, Lutz-Juergen, 34134 Kassel (DE); Reinbold, Christoph, 34560 Fritzlar (DE); Riepel, Dirk, 37308 Heilbad Heiligenstadt (DE); Roick, Sebastian, 99817 Eisenach (DE)
(74) Vertreter: Tergau & Walkenhorst Intellectual Property GmbH

(57) **Zusammenfassung**

Die Erfindung betrifft ein Verfahren zur automatisierten Endabfertigung von gleich- oder verschiedenartige Infusionspumpen (4) in einem Endabfertigungssystem (2) mit einer Mehrzahl von über ein Transportsystem (16) für die Infusionspumpen (4) miteinander verbundenen, zum konsekutiven Durchlauf vorgesehenen Stationen (14) und mit einer die Stationen (14) und das Transportsystem (16) steuernden Systemsteuerung (18), wobei jede Infusionspumpe (4) eine integrierte Pumpensteuerung (6) mit einem programmierbaren Speicher (8) für Betriebssoftware und/oder Konfigurationsdaten aufweist und mit einem maschinenlesbaren eindeutigen Kennzeichen (10) versehen ist, wobei ferner
• eine der Stationen (14) eine Programmierstation (22) ist, in der die jeweilige Infusionspumpe (4) mit einer ihrem eindeutigen Kennzeichen (10) zugeordneten individuellen Betriebssoftware programmiert und/oder individuell konfiguriert wird, und
• zumindest eine der Stationen (14) eine der Programmierstation durchlaufmäßig nachgeschaltete Prüf- und/oder Kalibrierstation (24, 24', 24") ist, in der die jeweilige Infusionspumpe (4) einer ihrem eindeutigen Kennzeichen (10) zugeordneten individuellen Prüfung und/oder Kalibrierung unterzogen wird.

## Beschreibung

Die Erfindung betrifft ein Verfahren zur automatisierten Endabfertigung von Infusionspumpen. Die Erfindung betrifft ferner ein entsprechendes Endabfertigungssystem.

Bislang werden Infusionspumpen manuell an aufeinanderfolgenden Arbeitsplätzen sukzessive durch Hinzufügen von Baugruppen gefertigt. Nach der Fertigung werden die Infusionspumpen mit Software bespielt. Prinzipiell könnten individuelle Konfigurationen an den Infusionspumpen vorgenommen werden, wie beispielsweise
- Voreinstellung der Sprache,
- Hinterlegung bestimmter Datensätze über zu nutzende Einmalartikel,
- Hinterlegung einer digitalen Medikamentenbibliothek.

In der Praxis wird dies im manuellen Produktionsablauf jedoch nicht gemacht, da die dazu erforderlichen Informationen in der Regel nicht am entsprechenden Arbeitsplatz vorliegen. Weiterhin wird die jeweilige Infusionspumpe in Bezug auf die Fördermenge und die Förderrate kontrolliert, und es werden Funktionsprüfungen und Fehlerprüfungen an der Infusionspumpe durchgeführt.

Die genannten Arbeitsschritte sind aufwendig sowie zeit- und kostenintensiv. Sie finden in der Regel außerhalb des Produktionsumfeldes statt. Aufgrund der Durchführung der vorgenannten Arbeiten durch unterschiedliche Personen des sogenannten Field Service kann das Ergebnis uneinheitlich sein.

Der Erfindung liegt die Aufgabe zugrunde, ein Verfahren zur automatisierten Endabfertigung von Infusionspumpen inklusive Kalibrierung und/oder Funktionsprüfung anzugeben, das sich sowohl für gleichartige Infusionspumpen, die erst durch unterschiedliche Konfiguration individualisiert werden, als auch für von vornherein unterschiedliche (Typen von) Infusionspumpen eignet, wobei die Kalibrier- und/oder Prüffunktionen individuell anpassbar sind. Des Weiteren soll ein zur Durchführung des Verfahrens geeignetes Endabfertigungssystem angegeben werden.

Die auf das Verfahren bezogene Aufgabe wird erfindungsgemäß gelöst durch ein Verfahren mit den Merkmalen des Anspruchs 1. Das zugehörige Endabfertigungssystem ist in Anspruch 15 spezifiziert.

Demnach manifestiert sich die Erfindung in einem Verfahren zur automatisierten Endabfertigung von gleich- oder verschiedenartige Infusionspumpen in einem Endabfertigungssystem mit einer Mehrzahl von über ein Transportsystem für die Infusionspumpen miteinander verbundenen, zum konsekutiven Durchlauf vorgesehenen Stationen und mit einer die Stationen und das Transportsystem steuernden Systemsteuerung, wobei jede Infusionspumpe eine integrierte Pumpensteuerung mit einem programmierbaren Speicher für Betriebssoftware und/oder Konfigurationsdaten aufweist und mit einem maschinenlesbaren eindeutigen Kennzeichen versehen ist, wobei ferner
- eine der Stationen eine Programmierstation ist, in der die jeweilige Infusionspumpe mit einer ihrem eindeutigen Kennzeichen zugeordneten individuellen Betriebssoftware programmiert und/oder individuell konfiguriert wird, und
- zumindest eine der Stationen eine der Programmierstation durchlaufmäßig nachgeschaltete Prüf- und/oder Kalibrierstation ist, in der die jeweilige Infusionspumpe einer ihrem eindeutigen Kennzeichen zugeordneten individuellen und spezifischen Prüfung und/oder Kalibrierung unterzogen wird.

Das zur Durchführung des erfindungsgemäßen Verfahrens vorgesehene Endabfertigungssystem (ATO - Assemble-to-order) umfasst mehrere Stationen, die sukzessive von den fertig vormontierten Infusionspumpen (Spritzenpumpen und/oder peristaltische Pumpen) durchlaufen werden. Dabei werden die Infusionspumpen mittels eines Transportsystems zwischen den Stationen transportiert. Jede Infusionspumpe umfasst ein maschinenlesbares eindeutiges Kennzeichen (UID - Unique Identifier). Vor oder beim Einfahren in die jeweilige Station wird das eindeutige Kennzeichen mittels eines zugehörigen Lesegerätes an der jeweiligen Station ausgelesen und überprüft. Mit dem Auslesen ist eine jeweilige Infusionspumpe individuell identifizierbar, und
1. es kann ein für die jeweilige Infusionspumpe hinterlegter Arbeitsauftrag (bestimmte Software oder Firmware installieren; bestimmte Konfiguration durchführen; bestimmte Funktionsprüfung durchführen; etc.) in der jeweiligen Station durchgeführt werden;
2. die durchgeführten Arbeitsaufträge (bestimmte Software oder Firmware installieren; bestimmte Konfiguration durchführen; bestimmte Funktionsprüfung durchführen; etc.) können digital dokumentiert werden.

In der den Prüf- und/oder Kalibrierstationen vorgeschalteten Programmierstation wird die jeweilige Infusionspumpe mit einer individuellen Betriebssoftware programmiert und/oder individuell konfiguriert. Nach dem Aufspielen der Betriebssoftware und deren Konfiguration ist die Pumpensteuerung der jeweiligen Infusionspumpe einem individuellen Prüf- oder/oder Kalibrierprozess in nachgelagerten Stationen zugänglich, der den Besonderheiten der individuellen, gerätespezifischen Ausstattung und/oder Konfiguration Rechnung trägt. Durch die gemäß der eindeutigen Kennzeichnung auftragsbezogene Anpassung des Prozesses ist praktisch eine automatisierte Gesamtsimulation eines späteren Produktivbetriebes an einem individualisierten Infusionsarbeitsplatz möglich.

In einer vorteilhaften Variante weist die jeweilige Infusionspumpe ein pumpenseitiges Schnittstellenmodul auf, welches in Verbindung mit zumindest einem stationsseitigen Schnittstellenmodul eine Schnittstelle zur Kommunikation zwischen Pumpensteuerung und Systemsteuerung ausbildet, wobei die Systemsteuerung über die Schnittstelle die Pumpensteuerung anspricht und eine Anzahl von Prüf- oder Betriebsroutinen der Infusionspumpe auslöst, die von der Prüf- und/oder Kalibrierstation überwacht werden. Bei der Schnittstelle zwischen Pumpensteuerung und Systemsteuerung kann es sich insbesondere um eine Infrarot- und/oder WiFi-Schnittstelle und/oder Bluetooth-Schnittstelle handeln.

Jede Station kann mit einem eigenem stationsseitigen Schnittstellenmodul ausgestatte sein. Dadurch ist es möglich, die jeweilige Infusionspumpe während des Transports zwischen den Stationen datenseitig von der Systemsteuerung zu entkoppeln und beim Einlaufen in die jeweilige Station mit der Systemsteuerung zu verbinden.

Die Systemsteuerung kann in eine der Stationen integriert sein, ist jedoch typischerweise baulich ausgelagert und zweckmäßigerweise mit den Stationen über geeignete Datenleitungen oder auf sonstige Weise datenmäßig, d. h. im Hinblick auf einen Datenaustausch, verbunden. Die einzelnen Stationen können für einen von der Systemsteuerung teilweise unabhängigen, autonomen oder teilautonomen Betrieb ausgelegt sein.

Vorteilhafterweise umfasst die in der Programmierstation vorgenommene Programmierung und/oder Konfiguration ein oder mehrere der folgenden Aktionen: Individuelle, dem eindeutigen Kennzeichen der jeweiligen Infusionspumpe zugeordnete Bereitstellung oder Einstellung einer Medikamentenbibliothek und/oder einer Einmalartikelbibliothek, Einstellung einer WiFi-Schnittstelle, Spracheinstellung.

Besonders bevorzugt ist es, wenn die Programmierstation eine Mehrzahl von nebeneinander angeordneten, jeweils eine Mehrzahl von jeweils eine Infusionspumpe aufnehmenden Programmiernestern aufweist, wobei alle Programmiernester eines Strangs durch einen gemeinsamen Computer gesteuert werden, der seinerseits durch die Systemsteuerung gesteuert wird. Diese Ausgestaltung trägt der Tatsache Rechnung, dass ein Programmiervorgang unterschiedlich lange dauern kann, beispielsweise zwischen 15 und 60 Minuten. Während ein Programmiernest noch belegt sein kann, kann ein anderes Programmiernest bereits freigeräumt und mit der nächsten Infusionspumpe belegt werden. Durch die mehreren Stränge wird eine Redundanz und somit Ausfallsicherheit gewährleistet.

Weiterhin ist es bevorzugt, dass die jeweilige Infusionspumpe ein resistives Touchdisplay aufweist, wobei eine der Stationen eine Display-Kalibrierstation ist, in der das Touchdisplay mittels eines Kamerasystems und eines gefederten Fingers inklusive Kraftsensor kalibriert. Dies geschieht beispielsweise, indem das Kamerasystem auf einem Roboter installiert ist und die angezeigten Kalibrierpunkte auf dem Display erfasst. Daraufhin werden die Verfahrwege des Roboters angepasst. Der Roboter fährt auf die Zielkoordinate und erhöht den Druck auf das Display, solange bis eine Kraft von festgelegter Größe über den Kraftsensor detektiert wird.

Optional werden in einer zugeordneten Station die LED-Signale der Infusionspumpe, der korrekte Verbau einer mechanischen Verriegelung und der PatientenAbleitstrom geprüft.

In besonders vorteilhafter Ausgestaltung umfassen die das Endabfertigungssystem durchlaufenden Infusionspumpen sowohl Spritzenpumpen als auch peristaltische Pumpen, wobei nach Art eines verzweigten Transportweges die Spritzenpumpen eine Prüf- und/oder Kalibrierstation für Spritzenpumpen und die peristaltischen Pumpen eine Prüf- und/oder Kalibrierstation für peristaltische Pumpen durchlaufen. Anschließend können die Transportwege wieder zusammengeführt werden und weitere gemeinsam durchlaufene Stationen umfassen.

Vorteilhafterweise werden in der Prüf- und/oder Kalibrierstation für Spritzenpumpen eine oder mehrere der folgenden Aktionen durchgeführt: Einlegen oder Entnehmen eines Kalibrierzylinders oder eines Prüfzylinders, Aufnehmen von Kraft-Weg-Sensorik, Prüfen von Spritzendetektion, Prüfen von einer Halte- und/oder Verriegelungsmechanik für Spritzen, Prüfung und/oder Kalibrierung eines Antriebskopfes.

In der Prüf- und/oder Kalibrierstation für peristaltische Pumpen hingegen werden vorteilhafterweise eine oder mehrere der folgenden Aktionen durchgeführt: Förderratenmessung und/oder Durchflussprüfung mittels Förderratensensorik, insbesondere Coriolissensorik, Druckprüfung der Peristaltik mittels Drucksensorik, Prüfen von Sicherheitsmechanismen nach Öffnen einer Gehäuseklappe und/oder im Fall von Schlauchdefekten, Prüfung eines Luftsensors.

In zweckmäßiger Ausgestaltung ist eine der Stationen, vorzugsweise am Ende des Transportweges, eine Labelingstation, in der eine individuelle Beschriftung der jeweiligen Infusionspumpe erfolgt.

Bei geeigneter Nachverfolgung der Transportwege und des Routings zwischen den Stationen mag es ausreichen, das eindeutige Kennzeichen der jeweiligen Infusionspumpe einmal zu Beginn des Transports auszulesen. Sicherer ist es jedoch, wenn zumindest in einigen, vorzugsweise in jeder der Stationen das eindeutige Kennzeichen maschinell ausgelesen und von der Systemsteuerung überprüft wird.

In vorteilhafter Ausgestaltung ist das eindeutige Kennzeichen der jeweiligen Infusionspumpe durch einen RFID-Transponder (Chip) implementiert, der beispielsweise im Gehäuse oder auf einer Leiterplatte der Infusionspumpe angeordnet ist. Alternative und/oder zusätzliche Mittel zur eindeutigen Kennzeichnung und Identifizierung der einzelnen Infusionspumpen sind möglich.

Zweckmäßigerweise ist das Transportsystem für die Infusionspumpen zwischen den Stationen schienengestützt. Beispielsweise können die Infusionspumpen in entsprechenden schienengeführten Gondeln, Gestellen oder Transportkörben gehalten werden, was bei einfach und kostengünstig gehaltener Ausgestaltung eine weitgehende Anpassung an unterschiedliche Formate von Infusionspumpen und durch Einsatz von Weichen eine hohe Flexibilität hinsichtlich der Transportwege ermöglicht. Verschiedenartige Infusionspumpen sind auf gleichen Gestellen befestigbar, wodurch ein einheitliches Transportsystem für alle Pumpen nutzbar ist und somit variabel und günstig herstellbar und betreibbar ist. Der Einsatz von Roboterarmen ist insbesondere für Teilabschnitte eine sinnvolle Option.

Von besonderem Vorteil ist es, wenn die Systemsteuerung die von ihr gesteuerten Vorgänge digital dokumentiert oder protokolliert, insbesondere in Form eines Herstellberichts für die jeweilige Infusionspumpe.

Ein automatisiertes Endabfertigungssystem gemäß der Erfindung für gleich- oder verschiedenartige Infusionspumpen weist eine Mehrzahl von über ein Transportsystem für die Infusionspumpen miteinander verbundenen, zum konsekutiven Durchlauf vorgesehenen Stationen und eine die Stationen und das Transportsystem steuernde Systemsteuerung auf, wobei jede Infusionspumpe eine integrierte Pumpensteuerung mit einem programmierbaren Speicher für Betriebssoftware und/oder Konfigurationsdaten aufweist und mit einem maschinenlesbaren eindeutigen Kennzeichen versehen ist, wobei ferner
- eine der Stationen eine Programmierstation ist, die dazu eingerichtet ist, die jeweilige Infusionspumpe mit einer ihrem eindeutigen Kennzeichen zugeordneten individuellen Betriebssoftware zu programmieren und individuell zu konfigurieren, und
- zumindest eine der Stationen eine der Programmierstation durchlaufseitig nachgeschaltete Prüf- und/oder Kalibrierstation ist, die dazu eingerichtet ist, die jeweilige Infusionspumpe einer ihrem eindeutigen Kennzeichen zugeordneten individuellen und spezifischen Prüfung und/oder Kalibrierung zu unterziehen.

Das für das Verfahren Gesagte lässt sich sinngemäß auf die Vorrichtung bzw. das Endabfertigungssystem anwenden, so dass an dieser Stelle Wiederholungen verzichtbar sind. Insbesondere können Vorrichtungs- und/oder Verfahrensmerkmale der Ansprüche 1 bis 14 auch Merkmale des in Anspruch 15 spezifizierten Endabfertigungssystems für Infusionspumpen sein.

Das erfindungsgemäße System und das zugehörige Verfahren ermöglichen zusammenfasend eine effiziente und zuverlässige Lösung zur Bereitstellung von kundenindividuellen ready-for-use Infusionspumpen. Dies umfasst die kundenindividuelle Bereitstellung und Einstellung der Medikamentenbibliothek, Konfigurationsdaten, Einmalartikelbibliothek (z. B. nutzbare Spritzen), WiFi-Einstellung, Spracheinstellung und weiteres. Durch die beschriebene Systemarchitektur ist insbesondere durch die datenmäßige Kopplung der Systemsteuerung an ein Fertigungsmanagementsystem (MES - Manufacturing Execution System) ein hoher Grad an Automatisierung bei zugleich hohem Grad an Individualisierung erreichbar.

Ein Ausführungsbeispiel der Erfindung wird nachfolgend anhand einer Zeichnung erläutert.
- FIG. 1: zeigt eine schematische Übersicht über ein Endabfertigungssystem für Infusionspumpen
- FIG. 2: zeigt eine Infusionspumpe in rein schematischer Darstellung.

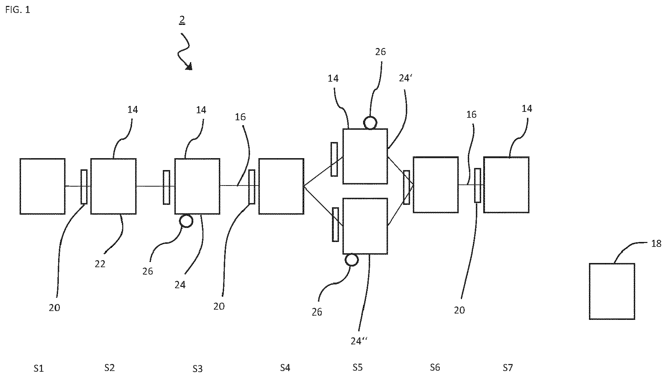

Das in FIG. 1 rein schematisch dargestellte Endabfertigungssystem 2 dient zur automatisierten Endabfertigung inklusive Kalibrierung und Funktionsprüfung von Infusionspumpen 4, und zwar sowohl Spritzenpumpen als auch peristaltischen Pumpen.

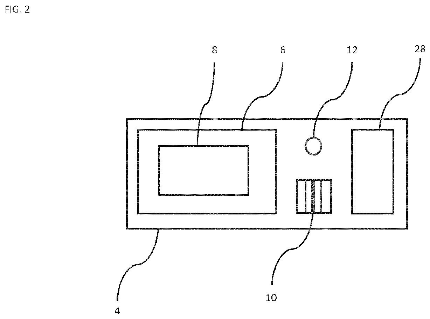

Jede der vom Endabfertigungssystem 2 bearbeiteten, in FIG. 2 rein schematisch dargestellten Infusionspumpen 4 weist eine integrierte Pumpensteuerung 6 mit einem programmierbaren Speicher 8 für Betriebssoftware und/oder Konfigurationsdaten auf und ist mit einem maschinenlesbaren eindeutigen Kennzeichen 10 versehen, hier im Beispiel in Gestalt eines RFID-Transponders, der beispielsweise auf einer Leiterpatte der Infusionspumpe 4 fest verankert ist. Ferner weist jede Infusionspumpe 4 ein pumpenseitiges Schnittstellenmodul 12 auf, dessen Funktion weiter unten erläutert wird.

Das Endabfertigungssystem 2 umfasst eine Mehrzahl von Stationen 14, in denen Arbeitsschritte zur Endabfertigung inklusive Kalibrierung und Funktionsprüfung der Infusionspumpen 4 automatisiert durchgeführt werden. Die Stationen 14 sind über ein schienengestütztes Transportsystem 16 derart miteinander verbunden, dass die Infusionspumpen 4 die Stationen 14, oder zumindest eine Teilmenge davon, nacheinander durchlaufen. Die einzelnen Stationen 14 und das Transportsystem 16 werden dabei von einem Zentralrechner einer Systemsteuerung 18 gesteuert.

Den einzelnen Stationen 14 (oder zumindest einigen davon) ist ein Lesegerät 20 für das eindeutige Kennzeichen 10 der aktuell zu bearbeitenden Infusionspumpe 4 vorgeschaltet oder integriert, hier im Beispiel ein RFID-Lesegerät. Das Lesegerät 20 ist datentechnisch mit der Systemsteuerung 18 verbunden. Dadurch ist eine Identifizierung der aktuell in die jeweilige Station 14 einfahrenden oder eingefahrenen Infusionspumpe 4 ermöglicht, was wiederum eine individuelle Endfertigung, Kalibrierung und/oder Funktionsprüfung mittels entsprechender Ansteuerung der Station 14 durch die Systemsteuerung 18 gestattet.

Eine der Stationen 14 ist eine Programmierstation 22, in der die jeweilige Infusionspumpe 4 mit einer ihrem eindeutigen Kennzeichen 10 zugeordneten individuellen Betriebssoftware programmiert und/oder individuell konfiguriert wird.

Zumindest eine der Stationen 14 ist eine der Programmierstation durchlaufmäßig nachgeschaltete Prüf- und/oder Kalibrierstation 24, 24`, 24", in der die jeweilige Infusionspumpe 4 einer ihrem eindeutigen Kennzeichen 10 zugeordneten individuellen Prüfung und/oder Kalibrierung unterzogen wird.

Dies geschieht bevorzugt in der Weise, dass das Schnittstellenmodul 12 der jeweiligen Infusionspumpe 4 mit einem stationsseitigen Schnittstellenmodul 26 eine Schnittstelle zur Kommunikation zwischen Pumpensteuerung 6 und Station 14 und damit auch (über die Station 14) zwischen Pumpensteuerung 6 und Systemsteuerung 18 ausbildet. Das genannte stationsseitige Schnittstellenmodul 26 kann an der jeweiligen Station 14 individuell ausgebildet sein. Alternativ ist auch eine mehrere Stationen 14 überspannendes systemseitiges Schnittstellenmodul 26 denkbar. Das heißt zusammenfassend, die Systemsteuerung 18 spricht über die Schnittstelle die Pumpensteuerung 6 an und löst eine Anzahl von Prüf- oder Betriebsroutinen der Infusionspumpe 4 aus, die wiederum von der Prüf- und/oder Kalibrierstation 24 überwacht werden.

Wie in FIG. 1 angedeutet, kann sich der Transportweg für verschiedenartige Infusionspumpen 4 verzweigen, etwa in dem Sinne, dass eine peristaltische Pumpe eine dezidierte Prüf- und/oder Kalibrierstation 24` für peristaltische Pumpen durchläuft, während eine Spritzenpumpe eine dezidierte Prüf- und/oder Kalibrierstation 24" für Spritzenpumpen durchläuft. Anschließend kann eine Wiedervereinigung der Transportwege erfolgen, so dass die Infusionspumpen 4 wieder gleiche Stationen 14 durchlaufen.

Zusätzlich zu den genannten automatisierte Stationen 14 können Stationen oder Arbeitsplätze vorhanden sein, in denen ein manueller Eingriff oder eine Sichtprüfung erfolgt. Diese sind aber für die hier beanspruchte technische Lehre nicht erforderlich.

Im Detail kann das Endabfertigungssystem 2 beispielsweise folgende Stationen umfassen:

### 1. Sichtprüfung I (S1)

### Prozess:

- Aufsetzen der Infusionspumpe auf das Transportsystem und Prüfung auf korrekte Position auf dem Transportgestell

### 2. Programmierstation (S2)

Die Programmierstation ist ausgestattet mit Programmiernestern, in denen die Infusionspumpen angedockt werden. Jedes Programmiernest umfasst einen Steckkontakt zur elektrischen Versorgung der angedockten Infusionspumpe. Mehrere Programmiernester sind übereinander in einem Strang angeordnet und jeder Strang ist mit einem separaten Computer gekoppelt, sodass eine Redundanz bei Ausfall eines Computers geschaffen ist. In der Programmierstation sind bevorzugt mehrere Stränge nebeneinander angeordnet, jeder Strang ist mit einem eigenen Computer gekoppelt. Dadurch wird eine Redundanz sichergestellt.

### Prozess:

- Identifizierung der Infusionspumpe (Typ, Modell, Seriennummer) mittels RFID oder anderem Kennzeichen
- Einfuhr und Positionierung auf Programmiernest (ein Turm/Strang umfasst einen Rechner und mehrere Programmiernester); die Anlage selbst kann mehrere Türme umfassen. Pumpen-Kommunikation via Infrarot und WiFi.
- Programmieren der im Fertigungsauftrag angegeben Software
- Überprüfung von Checksummen
- Programmierung des WiFi-Moduls der Infusionspumpe

### 3. Display-Kalibrierung (S3)

### Prozess:

- Identifizieren der Infusionspumpe
- Austausch der Prüfparameter für Pumpe mit dem übergeordneten System (MES - Manufacturing Execution System)
- Prüfung Patientenableitstrom
- Prüfung Bedienungsfelder - Abtastpunkte (Bedienungstaster); ggf. Kalibrierung von Touchdisplay mittels Kamerasystem und einem gefederten Finger inklusive Kraftsensor, um immer gleiche Kräfte auf das resistive Display aufzubringen.
- LED-Funktionstest mittels Kamera.

### 4. Sichtprüfung II (S4)

### Prozess:

- Identifizieren der Infusionspumpe
- Öffnen der Frontklappe

### 5. Prüf- und Kalibrierstation (S5)

Abhängig von der Infusionspumpenart (Infusomat = peristaltische Pumpe oder Perfusor = Spritzenpumpe) werden die Infusionspumpen in unterschiedliche Kalibrierungsstationen weitergeführt und in diesen jeweils automatisch und Zuhilfenahme von Prüfmitteln / Kalibriermitteln / Testmitteln kalibriert.

### 6. Sichtprüfungsstation III (S6)

### Prozess:

- Entnahme des Prüfmittels Prüfschlauchs
- Upload Qualitätszertifikat auf Pumpe
- Optional: Upload Medikamentendatenbank, Einmalartikelliste oder Konfigurationsdatei
- Pumpe in Auslieferungszustand versetzen

### 7. Labelingstation (S7)

### Prozess:

- Beschriftung des Typenschilds auf Infusionspumpe via Laser
- Überprüfung des Schilds
- Weitergabe an Verpackungsstation

### Bezugszeichenliste

- 2: Endabfertigungssystem
- 4: Infusionspumpe
- 6: Pumpensteuerung
- 8: Speicher
- 10: eindeutiges Kennzeichen
- 12: Schnittstellenmodul
- 14: Station
- 16: Transportsystem
- 18: Systemsteuerung
- 20: Lesegerät
- 22: Programmierstation
- 24: Prüf- und/oder Kalibrierstation
- 24`: Prüf- und/oder Kalibrierstation
- 24": Prüf- und/oder Kalibrierstation
- 26: Schnittstellenmodul
- 28: Touchdisplay

- S1...S7: Stationen

## Patentansprüche

1. Verfahren zur automatisierten Endabfertigung von gleich- oder verschiedenartige Infusionspumpen (4) in einem Endabfertigungssystem (2) mit einer Mehrzahl von über ein Transportsystem (16) für die Infusionspumpen (4) miteinander verbundenen, zum konsekutiven Durchlauf vorgesehenen Stationen (14) und mit einer die Stationen (14) und das Transportsystem (16) steuernden Systemsteuerung (18), wobei jede Infusionspumpe (4) eine integrierte Pumpensteuerung (6) mit einem programmierbaren Speicher (8) für Betriebssoftware und/oder Konfigurationsdaten aufweist und mit einem maschinenlesbaren eindeutigen Kennzeichen (10) versehen ist, wobei ferner
• eine der Stationen (14) eine Programmierstation (22) ist, in der die jeweilige Infusionspumpe (4) mit einer ihrem eindeutigen Kennzeichen (10) zugeordneten individuellen Betriebssoftware programmiert und/oder individuell konfiguriert wird, und
• zumindest eine der Stationen (14) eine der Programmierstation durchlaufmäßig nachgeschaltete Prüf- und/oder Kalibrierstation (24, 24`, 24") ist, in der die jeweilige Infusionspumpe (4) einer ihrem eindeutigen Kennzeichen (10) zugeordneten individuellen Prüfung und/oder Kalibrierung unterzogen wird.

2. Verfahren nach Anspruch 1, wobei die jeweilige Infusionspumpe (4) ein pumpenseitiges Schnittstellenmodul (12) aufweist, welches in Verbindung mit zumindest einem stationsseitigen Schnittstellenmodul (26) eine Schnittstelle zur Kommunikation zwischen Pumpensteuerung (6) und Systemsteuerung (18) ausbildet, und wobei die Systemsteuerung (18) über die Schnittstelle die Pumpensteuerung (6) anspricht und eine Anzahl von Prüf- oder Betriebsroutinen der Infusionspumpe (4) auslöst, die von der Prüf- und/oder Kalibrierstation (24, 24`, 24") überwacht werden.

3. Verfahren nach Anspruch 1 oder 2, wobei die in der Programmierstation (22) vorgenommene Programmierung und/oder Konfiguration ein oder mehrere der folgenden Aktionen umfasst: Individuelle, dem eindeutigen Kennzeichen (10) der jeweiligen Infusionspumpe (4) zugeordnete Bereitstellung oder Einstellung einer Medikamentenbibliothek und/oder einer Einmalartikelbibliothek, Einstellung oder Programmierung einer WiFi-Schnittstelle, Spracheinstellung.

4. Verfahren nach einem der vorhergehenden Ansprüche, wobei die Programmierstation (22) eine Mehrzahl von nebeneinander angeordneten, jeweils eine Mehrzahl von jeweils eine Infusionspumpe (4) aufnehmenden Programmiernestern aufweist, wobei alle Programmiernester eines Strangs durch einen gemeinsamen Computer gesteuert werden, der seinerseits durch die Systemsteuerung (18) gesteuert wird.

5. Verfahren nach einem der vorhergehenden Ansprüche, wobei die jeweilige Infusionspumpe (4) ein resistives Touchdisplay (28) aufweist, und wobei eine der Stationen (14) eine Display-Kalibrierstation ist, in der das Touchdisplay (28) mittels eines Kamerasystems und eines gefederten Fingers inklusive Kraftsensor kalibriert wird.

6. Verfahren nach einem der vorhergehenden Ansprüche, wobei die das Endabfertigungssystem (2) durchlaufenden Infusionspumpen (4) sowohl Spritzenpumpen als auch peristaltische Pumpen umfassen, und wobei nach Art eines verzweigten Transportweges die Spritzenpumpen eine Prüf- und/oder Kalibrierstation (24`) für Spritzenpumpen und die peristaltischen Pumpen eine Prüf- und/oder Kalibrierstation (24") für peristaltische Pumpen durchlaufen.

7. Verfahren nach Anspruch 6, wobei in der Prüf- und/oder Kalibrierstation (24`) für Spritzenpumpen eine oder mehrere der folgenden Aktionen durchgeführt werden: Einlegen oder Entnehmen eines Kalibrierzylinders oder eines Prüfzylinders, Aufnehmen von Kraft-Weg-Sensorik, Prüfen von Spritzendetektion, Prüfen von einer Halte- und/oder Verriegelungsmechanik für Spritzen, Prüfung und/oder Kalibrierung eines Antriebskopfes.

8. Verfahren nach Anspruch 6 oder 7, wobei in der Prüf- und/oder Kalibrierstation (24") für peristaltische Pumpen eine oder mehrere der folgenden Aktionen durchgeführt werden: Förderratenmessung und/oder Durchflussprüfung mittels Förderratensensorik, insbesondere Coriolissensorik, Druckprüfung der Peristaltik mittels Drucksensorik, Prüfen von Sicherheitsmechanismen nach Öffnen einer Gehäuseklappe und/oder im Fall von Schlauchdefekten, Prüfung eines Luftsensors.

9. Verfahren nach einem der vorhergehenden Ansprüche, wobei eine der Stationen (14), vorzugsweise am Ende des Transportweges, eine Labelingstation ist, in der eine individuelle Beschriftung der jeweiligen Infusionspumpe (4) erfolgt.

10. Verfahren nach einem der vorhergehenden Ansprüche, wobei zumindest in einigen, vorzugsweise in jeder der Stationen (14) das eindeutige Kennzeichen (10) maschinell ausgelesen und von der Systemsteuerung (18) überprüft wird.

11. Verfahren nach einem der vorhergehenden Ansprüche, wobei das eindeutige Kennzeichen (10) der jeweiligen Infusionspumpe durch einen RFID-Transponder implementiert ist.

12. Verfahren nach einem der vorhergehenden Ansprüche, wobei die Schnittstelle zwischen Pumpensteuerung (6) und Systemsteuerung (18) eine Infrarot- und/oder WiFi-Schnittstelle ist.

13. Verfahren nach einem der vorhergehenden Ansprüche, wobei das Transportsystem (16) schienengestützt ist.

14. Verfahren nach einem der vorhergehenden Ansprüche, wobei die Systemsteuerung (18) die von ihr gesteuerten Vorgänge digital dokumentiert oder protokolliert, insbesondere in Form eines Herstellberichts für die jeweilige Infusionspumpe (4).

15. Automatisiertes Endabfertigungssystem (2) für gleich- oder verschiedenartige Infusionspumpen (4) mit einer Mehrzahl von über ein Transportsystem (16) für die Infusionspumpen (4) miteinander verbundenen, zum konsekutiven Durchlauf vorgesehenen Stationen (14) und mit einer die Stationen (14) und das Transportsystem (16) steuernden Systemsteuerung (18), wobei jede Infusionspumpe (4) eine integrierte Pumpensteuerung (6) mit einem programmierbaren Speicher (8) für Betriebssoftware und/oder Konfigurationsdaten aufweist und mit einem maschinenlesbaren eindeutigen Kennzeichen (10) versehen ist, wobei ferner
• eine der Stationen (14) eine Programmierstation (22) ist, die dazu eingerichtet ist, die jeweilige Infusionspumpe (4) mit einer ihrem eindeutigen Kennzeichen (10) zugeordneten individuellen Betriebssoftware zu programmieren und/oder individuell zu konfigurieren, und
• zumindest eine der Stationen (14) eine der Programmierstation durchlaufseitig nachgeschaltete Prüf- und/oder Kalibrierstation (24, 24`, 24") ist, die dazu eingerichtet ist, die jeweilige Infusionspumpe (4) einer ihrem eindeutigen Kennzeichen (10) zugeordneten individuellen Prüfung und/oder Kalibrierung zu unterziehen.
